# EUROPEAN PATENT SPECIFICATION

(11) **EP 2 280 679 B1**
(45) Date of publication and mention of the grant of the patent: **08.08.2012**
(21) Application number: 09755669.0
(22) Date of filing: 27.05.2009
(51) Int. Cl.: A61F 13/49, A61F 13/15, B32B 37/14, B29C 65/00, B29C 65/52, B29C 69/00, B29C 65/78

(54) **METHODS AND APPARATUS FOR ATTACHING ELASTIC COMPONENTS TO ABSORBENT ARTICLES**
VERFAHREN UND VORRICHTUNG ZUR BEFESTIGUNG ELASTISCHER KOMPONENTEN AN SAUGFÄHIGEN ARTIKELN
PROCÉDÉS ET APPAREIL POUR FIXER DES COMPOSANTS ÉLASTIQUES À DES ARTICLES ABSORBANTS

(30) Priority: 27.05.2008 US 56131 P
(43) Date of publication of application: 09.02.2011
(73) Proprietor: The Procter & Gamble Company, Cincinnati, OH 45202 (US)
(72) Inventor: GILL, Nathan, Alan, Cincinnati OH 45244 (US); FOX, Jessica, Lynn, Cincinnati OH 45208 (US)
(74) Representative: Kremer, Véronique Marie Joséphine
(86) International application number: PCT/US2009/045219
(87) International publication number: WO 2009/146307

(56) References cited:
- EP-A- 0 443 244
- EP-A- 0 741 999
- US-A- 4 925 520
- US-A- 4 943 340
- US-A- 5 043 036
- US-A- 5 407 507
- US-A- 5 545 285
- US-A1- 2005 145 322

## Description

### FIELD OF THE INVENTION

The present disclosure relates to methods and apparatuses for manufacturing disposable absorbent articles, and more particularly, methods and apparatuses for attaching elastic components, such as waistbands, side panels, cuffs, or other component with cross direction stretch to disposable absorbent articles.

### BACKGROUND OF THE INVENTION

Along an assembly line, diapers and various types of other absorbent articles may be assembled by adding components to and otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. Webs of material and component parts used to manufacture diapers may include: backsheets, topsheet, absorbent cores, front and/or back ears, fastener components, and various types of elastic webs and components such as leg elastics, barrier leg cuff elastics, and waist elastics. Once the desired component parts are assembled, the advancing web(s) and component parts are subjected to a final knife cut to separate the web(s) into discrete diapers or other absorbent articles. The discrete diapers or absorbent articles may also then be folded and packaged.

Various methods and apparatuses may be used for attaching different components to the advancing web. US 5,043,036 discloses a method comprising stretching an elongate strip material between its edges to increase the width of the strip material, cutting the elongated material and applying it in spaced relationship along a moving substrate. US 5,407,507 discloses a method comprising cutting an unstretched elastic member from an elasticized web, stretching it in the cross-machine direction and combining the tensioned elastic member to a moving substrate.Some of methods and apparatuses relate to securing elastic bands, and more particularly, elastic waistbands to an advancing web. In some processes, elastic bands are adhered to an advancing web in a stretched condition. However, the configurations of some existing methods and apparatuses add cost and complexity to manufacturing processes. For example, some methods and apparatuses require a web to advance at relatively low speeds. Sometimes, the waistband material must be advanced in a first direction, stretched, rotated, and advanced in a second direction before being applied to an advancing web. In some instances, glue used to adhere the waist bands is applied intermittently, which results in inaccurate application, waste, and interference with other web handling equipment used in the manufacturing process.

### SUMMARY OF THE INVENTION

Aspects of the present disclosure involve methods for manufacturing absorbent articles, and more particularly, methods for applying elastic components to a moving substrate or web of material during the manufacture of disposable absorbent articles. Particular embodiments of methods of manufacture disclosed herein provide for the addition of elastic waistbands to various types of diaper configurations. While the present disclosure relates mainly to addition of waistbands to diapers, it is to be appreciated that the methods disclosed herein can also be applied to other elastic components used on diapers as well as other types of absorbent articles.

In one form, a method for applying elastic bands to a moving substrate includes the steps of: continuously feeding a first substrate of material in a machine direction at a first speed, the first substrate having a first surface disposed opposite of a second surface; continuously feeding a second substrate of material in the machine direction at a second speed, the second substrate having a first surface disposed opposite of a second surface; stretching the first substrate in a cross direction; rotating an anvil roll at a first rotational speed wherein an outer surface of the anvil roll travels at the first speed; feeding the first substrate onto the anvil roll such that the first surface of the first substrate is disposed on the outer surface of the anvil roll; maintaining the cross direction stretch of the first substrate while disposed on the outer surface of the anvil roll; applying glue to the second surface first substrate; cutting the stretched first substrate on the anvil roll into discrete stretched bands; and individually transferring the discrete stretched bands from the anvil roll onto the first surface of the second substrate such that the glue adheres the discrete stretched bands to the second substrate, and wherein the discrete stretched bands are displaced from each other in the machine direction along a length of the second substrate.

### BRIEF DESCRIPTION OF THE DRAWINGS

Fig. 1 is a schematic showing a first isometric view of an apparatus for attaching waist bands to an advancing substrate.
Fig. 2 is a schematic showing a second isometric view of the apparatus shown in Fig. 1.
Fig. 3 is a schematic showing a third isometric view of the apparatus shown in Fig. 1.
Fig. 4 is a side view of the apparatus shown in Fig. 1.
Fig. 4A is a side view of the apparatus shown in Fig. 1 with an oiler roll.
Fig. 5 is a detailed view of a bump roll transfer configuration shown in Fig. 3.
Fig. 6 is a detailed view of a tamper transfer configuration.
Fig. 7 is a detailed view of a roll to roll transfer configuration.
Fig. 8 is a top plan view of a disposable incontinent absorbent article that may utilize one or more elastic waist bands applied in accordance with the present disclosure.

### DETAILED DESCRIPTION OF THE INVENTION

The following term explanations may be useful in understanding the present disclosure:
"Absorbent article" is used herein to refer to consumer products whose primary function is to absorb and retain soils and wastes.
"Diaper" is used herein to refer to an absorbent article generally worn by infants and incontinent persons about the lower torso.
The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).
The term "disposed" is used herein to mean that an element(s) is formed (joined and positioned) in a particular place or position as a macro-unitary structure with other elements or as a separate element joined to another element.
As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.
The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a layer or layers or fibrous materials, films and foils such as plastic films or metallic foils that may be used alone or laminated to one or more web, layer, film and/or foil. As such, a web is a substrate.
The term "nonwoven" refers herein to a material made from continuous (long) filaments (fibers) and/or discontinuous (short) filaments (fibers) by processes such as spunbonding, meltblowing, and the like. Nonwovens do not have a woven or knitted filament pattern.
The term "machine direction" (MD) is used herein to refer to the direction of material flow through a process.
The term "cross direction" (CD) is used herein to refer to a direction that is generally perpendicular to the machine direction.
As used herein the term "stretchable" refers to materials which can stretch to at least an elongated length of 105% on the upcurve of the hysteresis test at a load of about 400 gm/cm. The term "non-stretchable" refers to materials which cannot stretch to at least 5% on the upcurve of the hysteresis test at a load of about 400 gm/cm.
The terms "elastic" and "elastomeric" as used herein refer to any material that upon application of a biasing force, can stretch to an elongated length of at least about 110% of its relaxed, original length (i.e. can stretch to 10 % more than its original length), without rupture or breakage, and upon release of the applied force, recovers at least about 40% of its elongation. For example, a material that has an initial length of 100 mm can extend at least to 110 mm, and upon removal of the force would retract to a length of 106 mm (40% recovery). The term "inelastic" refers herein to any material that does not fall within the definition of "elastic" above.
The term "extensible" as used herein refers to any material that upon application of a biasing force, can stretch to an elongated length of at least about 110% of its relaxed, original length (i.e. can stretch to 10 %), without rupture or breakage, and upon release of the applied force, shows little recovery, less than about 40% of its elongation.
The terms "activating", "activation" or "mechanical activation" refer to the process of making a substrate, or an elastomeric laminate more extensible than it was prior to the process.
"Live stretch" includes stretching elastic and bonding the stretched elastic to a substrate. After bonding, the stretched elastic is released causing it to contract, resulting in a "corrugated" substrate. The corrugated substrate can stretch as the corrugated portion is pulled to about the point that the substrate reaches at least one original flat dimension. However, if the substrate is also elastic, then the substrate can stretch beyond the relaxed length of the substrate prior to bonding with the elastic. The elastic is stretched at least 25% of its relaxed length when it is bonded to the substrate.

Aspects of the present disclosure involve methods for manufacturing absorbent articles, and more particularly, methods for applying discrete elastic components to a moving substrate or web of material during the manufacture of disposable absorbent articles. Particular embodiments of methods of manufacture disclosed herein provide for the addition of elastic waistbands to various types of diaper configurations. Elastic waistbands used on diapers may be configured to help optimize fit and comfort of a diaper on a wearer's body. In some embodiments, the waistband is made of a cross directional stretch material and may be disposed on the outside of the diaper. In other embodiments, the waist band may be disposed between substrates of material and may also be disposed on the inside of the diaper. The methods and apparatuses disclosed herein provide simplified processes as well as other beneficial results associated with placing cross directional stretch material waistbands on diapers. For example, as opposed to applying a cross directional stretch to individual waistbands, the disclosed processes and apparatus apply a cross directional stretch to a continuous substrate of waistband material to reduce necking in the stretched waistbands. In another example, to reduce issues with unglued edges and exposed glue, the present methods and apparatus apply adhesive continuously to the stretched waistband substrate, rather than intermittently applying glue to a moving substrate. While the present disclosure relates mainly to addition of elastic components such as waistbands to diapers, it is to be appreciated that the methods and apparatuses disclosed herein can also be applied to other elastic components used on diapers as well as other types of absorbent articles. For example, elastic components can include pre-stretched ears or side panels, cuffs placed in a side saddle process where the product's waist regions are parallel to the machine direction, or other components requiring stretch in the cross machine direction. In other applications, the elastic components can comprise elastic topsheets for a diaper cut from a substrate stretched in the cross-direction before being connected with other diaper components, such as a backsheet.

In some process configurations according to the present disclosure, elastic components, such as waistbands, are cut from a first substrate of material and adhered to a second substrate of material during assembly of an absorbent article, such as a diaper. Particular aspects of methods discussed herein may include the steps of feeding the first substrate of material in a machine direction at a first speed and continuously feeding the second substrate of material in the machine direction at a second speed. The second speed may be greater than the first speed, and as such, the first substrate may be advancing slower than the second substrate. It is also to be appreciated that in some configurations, the first substrate may advance at the same speed as the second substrate, while in other configurations, the first substrate may advance at a faster speed than the second substrate. The first substrate of material may then be stretched in the cross direction and fed onto an anvil roll. The anvil roll may be rotated at a velocity such that an outer surface of the anvil roll travels at the same speed as the first substrate. Once disposed on the outer surface of the anvil roll, glue or adhesive may be applied to the first substrate. Subsequent to applying the adhesive, the first substrate is cut in the cross direction into discrete elastic components, also referred to herein as elastic strips, patches, panels, and bands, while maintained in a stretched state on the anvil roll. It is appreciated that the cross directional cut may be in the form of a straight line, a curved line cut, jagged lines, or combinations thereof. From the anvil roll, the discrete elastic components are then individually transferred onto the second substrate such that the glue adheres the discrete stretched bands to the second substrate. The speed mismatch between the first substrate and second substrate results in the discrete stretched bands being displaced from each other in the machine direction along a length of the second substrate. As discussed below, the method steps disclosed herein can be carried out in different ways by various types of mechanisms.

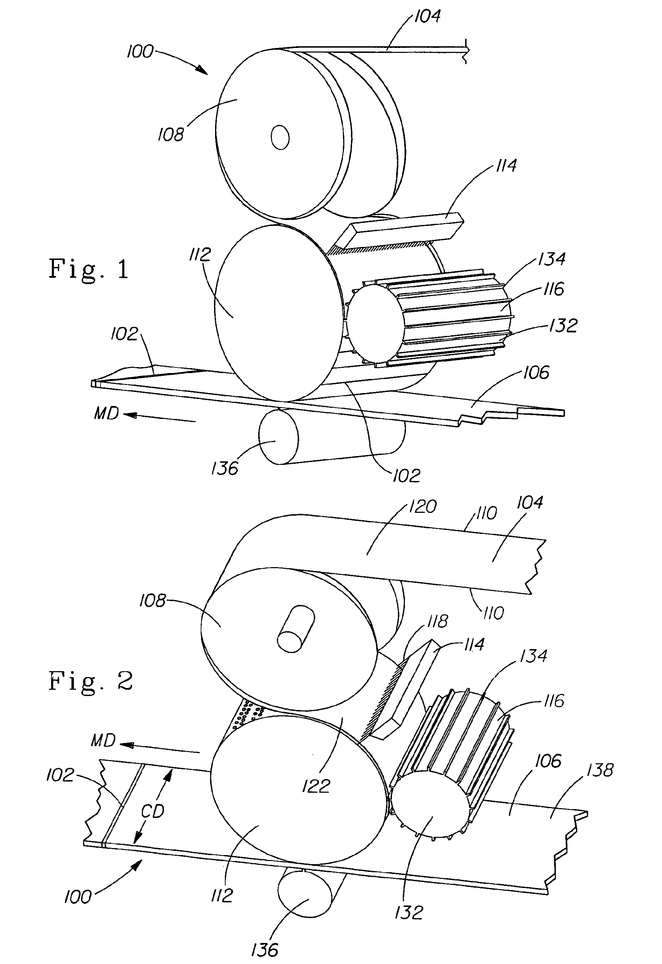

Figs. 1-4 show various schematic views of an apparatus 100 configured to perform various method steps for attaching stretched elastic waistbands 102 cut from a first substrate of material 104 to a second substrate of material 106. As shown in Figs. 1-4, the first substrate of material 104 and the second substrate of material 106 both travel in a machine direction (MD). In some embodiments, the first substrate 104 travels at a first speed and the second substrate 106 travels at a second speed. As discussed in more detail below, the second speed may be faster than the first speed. It is to be appreciated that the second substrate of material 106 can be utilized in various components of an assembled absorbent article, such as for example, a topsheet or backsheet of a diaper. Various types of suitable materials for various diaper components are discussed in more detail below with reference to an example diaper embodiment. It should also be appreciated that the first substrate of material 104 can be constructed from various types of materials. For example, the first substrate can include a combination of laminated substrates such as stretch films, poly films, and nonwovens. In some examples, the first substrate is constructed from a single stretch or poly film. In yet other examples, the first substrate is constructed from a single layer nonwoven or activated nonwoven.

As shown in Figs. 1-4, the first substrate 104 engages a spreader mechanism 108 that stretches the first substrate 104 in a cross direction (CD). Various types of spreader mechanisms 108 can be used to stretch the first substrate 104. Examples of spreader mechanisms 108 include diverging conveyors and canted rolls. The spreader mechanism 108 may also utilize vacuum, friction, belts, and/or fingers to hold the first substrate in a stretched configuration. Other spreading mechanisms may include ARCO rolls and bow bars. Although not typically used to stretch a substrate beyond a free width, ARCO rolls and bow bars may be effective at removing wrinkles from the first substrate. It is to be appreciated that the methods and apparatuses described herein can be configured to be compatible with a wide range of material widths in the cross direction. In some embodiments, the first substrate 104 may have an unstretched cross directional width of about 100 mm to about 250 mm. The spreader mechanism 108 can be configured to impart various degrees of stretch to the first substrate. For example, the spreader may be configured to impart about 40% to about 100% cross directional stretch to the first substrate. In such a configuration, a first substrate having an unstretched cross directional width of about 100 mm to about 250 mm would be stretched in a cross directional width to about 140 mm to about 500 mm.

Depending on the how the spreader mechanism 108 engages the first substrate 104, portions of the first substrate adjacent longitudinal edges 110 thereof may not be stretched. For example, if the spreader mechanism 108 grabs or holds the first substrate 104 adjacent the opposing longitudinal edges 110 while stretching the first substrate in the cross direction, small portions or areas of the first substrate where the spreader mechanism is holding or grabbing the first substrate may not be stretched in cross direction. As such, in some embodiments where it is desirable to utilize only the stretched portions of the first substrate, the aforementioned unstretched portions of the first substrate may be trimmed or otherwise removed from the final product.

From the spreader mechanism 108, the first substrate 104 is transferred to an anvil roll 112. As discussed in more detail below, the anvil roll 112 rotates and maintains advancement of the first substrate 104 and cross directional stretch in the first substrate. While engaged with the anvil roll 112, the first substrate 104 is advanced past an adhesive applicator 114 and a cutter 116. More particularly, the adhesive applicator 114 applies glue 118 to the first substrate 104, and the cutter 116 cuts the first substrate 104 into individual elastic components, also referred to herein as elastic strips, panels, patches, and bands and as shown in Figs. 1-4, in the form of elastic waistbands 102. Although the first substrate 104 is cut into individual elastic waistbands 102, the anvil roll 112 maintains the cross directional CD stretch in the waistbands 102 that was maintained in the first substrate 104 before being cut.

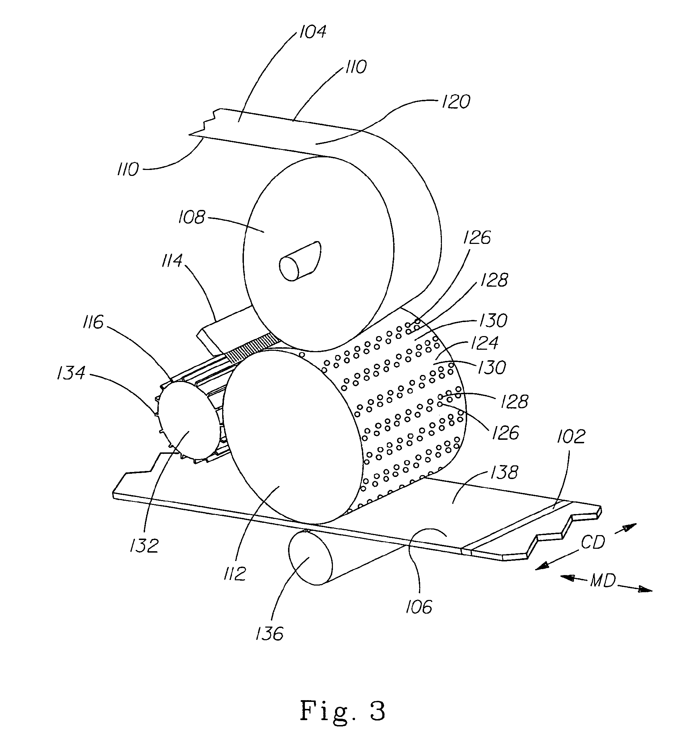

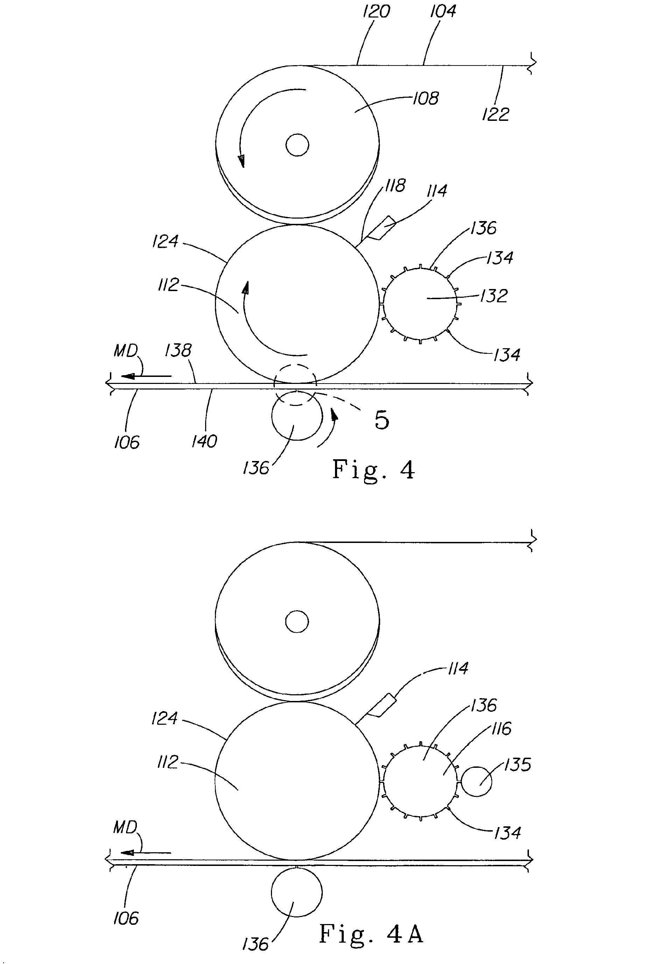

As shown in Figs. 1-4, a first surface 120 of the first substrate 104, which is disposed opposite a second surface 122, is advanced on an outer surface 124 of the anvil roll 112 from the spreader mechanism 108. The anvil roll 112 may rotate in a direction shown in Fig. 4 at a rotational speed that results in a speed of the outer surface 124 of the anvil roll 112 that is about equal to the first speed of the first substrate 104. As previously mentioned, the anvil roll 112 may be configured to maintain the cross directional CD stretch in the first substrate imparted by the spreader mechanism 108. In some embodiments, the anvil roll 112 is configured with a vacuum that holds the first substrate 104 in position on the outer surface 124 and maintains the cross directional stretch in the first substrate. For example, as shown in Fig. 3, a plurality of vacuum holes 126 may be located in the outer surface 124 of the anvil roll 112. The vacuum holes 126 may be disposed in a plurality of groups on the outer surface. Each group of vacuum holes may be located in vacuum areas 128 defined by a length and a width, wherein the length is defined by a portion of the circumference of the outer surface 124 of the anvil roll 112 and the width is defined by a portion of the cross directional CD width of the anvil roll. The vacuum areas may be radially separated by a plurality of anvil surfaces 130 on the outer surface 124 of the anvil 112. The number of anvil surfaces 130 and vacuum areas 128 can vary depending on the desired implementation. In operation, the vacuum holes 126 engage the first surface 120 of the first substrate 104 and hold the first substrate 104 in contact with the outer surface 124 of the anvil roll 112 while maintaining the cross directional stretch in the first substrate.

Once advanced onto the outer surface 124 of the anvil roll 112, the first substrate 104 moves past the adhesive applicator 114, as shown in Figs. 1-4. The adhesive applicator 114 deposits glue 118 on the second surface 122 of the first substrate 104. In some embodiments, the adhesive applicator 104 is configured to continuously apply glue 118 to the second surface 122 of the first substrate 104. It is to be appreciated that the glue can also be applied various different patterns. In some embodiments, the glue 118 may be applied in the form of a spray. Various glue application configurations can be used. For example, in some embodiments, contact glue applicators (i.e. applicators that touch the substrate during glue application) such as slot coater glue applicators may be used. In other embodiments, non-contact glue applicators (i.e. applicators that do not touch the substrate during glue application) such as spiral, meltblown, and omega glue applicators may be used. In some embodiments, the glue is elastic when dry, which allows the elastic waistband 102 to expand and contract after application. To help reduce contamination of the anvil roll in some embodiments, glue is not applied to areas disposed adjacent the outer edges 110 of the first substrate 104.

It should also be appreciated that some embodiments of the methods and apparatuses disclosed herein may be configured to add glue to the first substrate in different locations along the process. For example, in some embodiments, glue may be added to the first substrate before the first substrate engages the spreader mechanism. In other embodiments, the first substrate is pre-glued, so no additional glue is required to be added during the process.

After glue 118 has been applied to the first substrate 104, the first substrate 104 is cut into discrete elastic components, also referred to herein as elastic strips, panels, patches, and bands 102. As shown in Figs. 1-4, the cutter 116 engages the first substrate 104 on the rotating anvil roll 112. It is to be appreciated that various types of mechanisms can be utilized as the cutter 116. For example, the cutter 116 may be in form of a rotating knife roll 132, as shown in Figs. 1-4. In some embodiments, the knife roll 132 is configured as a die cutter having one or more die or blades 134 protruding from the outer surface of the knife roll 132. It is to be appreciated that the cutter 116 can be configured with various numbers of blades or die. It is also to be appreciated that the die extend in the cross direction and can have cutting edges of different shapes and sizes, such as straight, curved, jagged, or combinations thereof. In some embodiments, the blades have straight edges which result in straight waistband shapes. In other embodiments, the blades have curved edges which result in curved waistband shapes. As the knife roll 132 rotates, the die 134 contact and cut the first substrate 104 against the anvil surfaces 130 on the anvil roll 112. It is to be appreciated that the rotational speed, size, die shape, and numbers of die may vary depending on the desired implementation. For example, in one embodiment, the surface speed of the knife roll 132 is set to match the surface speed of the anvil roll 112. In another example, one embodiment may utilize a knife roll with a 100 mm diameter. As previously mentioned, the anvil roll 112 maintains the cross directional stretch in the individual waistbands 102 that was originally imparted to the first substrate 104.

As shown in Fig. 4A, an oiler roll 135 may be located adjacent to the knife roll. The oiler roll 135 applies oil to the blades or die 134 on the knife roll 132. The oil may help provide a relatively smoother cutting surface by filling in worn areas of the blade, resulting in a more uniform cut so the cutting surface is smooth. The oil may also help clean glue from the knife blade.

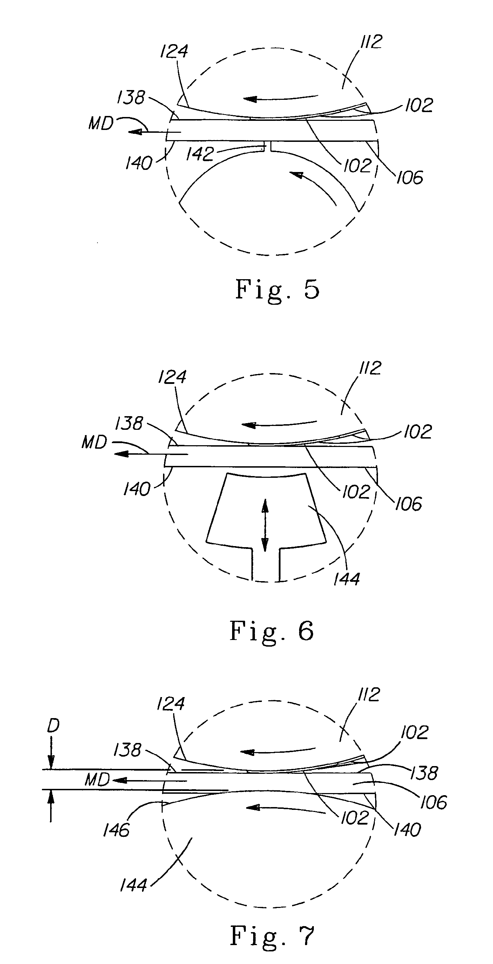

From the anvil roll 112, the discrete elastic waistbands 102 are transferred to the second substrate 106. As discussed above, the first substrate 104 and outer surface 124 of the anvil roll 112 move at the first speed, whereas the second substrate 106 moves at the second speed. In some embodiments, the second speed is greater than the first speed. As such, the individual elastic waistbands 102 are spaced from each other in the machine direction on the second substrate 106. As shown in Figs. 4 and 5, a rotating bump roll 136 may be used to help transfer the individual waistbands 102 from the anvil roll 112 to the second substrate 106. In the transfer process, the second substrate 106 advances between the anvil roll 112 and the bump roll 136 such that a first surface 138 of the second substrate 106 is adjacent the anvil roll 112 and an oppositely disposed second surface 140 of the second substrate 106 is adjacent the bump roll 136. The bump roll 136 may include one or more protrusions 142 that intermittently engage the second surface 140 of the second substrate 106 as the bump roll 136 rotates. Thus, when the protrusions 142 engage the second surface 140 of the second substrate 106, the first surface 138 of the second substrate 106 is moved into contact with one of the waistbands 102 on the anvil roll 112. At this time, glue 118 on the waistband 102 adheres the waistband to the first surface 138 of the second substrate 106. As the bump roll 136 continues to rotate, the protrusion 142 disengages the second substrate 106, and the second substrate 106 moves back away from the anvil roll 112 along with the waistband 102 adhered thereto. The process is repeated each time a protrusion on the bump roll engages the second substrate. It is to be appreciated that the bump roll 136 can be configured with various numbers of protrusions, shapes, sizes, and rotational speeds depending on the desired implementation. For example, in some embodiments, the surface speed of the bump roll 136 matches the second speed of the second substrate 106. In other examples, the bump roll protrusion 142 is configured with a relatively flat contact surface adapted to contact the second substrate. In embodiments that utilize a curved waistband, the protrusions may be configured with a pattern on the contact surface that matches the curved waistband shape.

It should also be appreciated that the apparatuses and methods described herein can utilize various types of transfer configurations other than the bump roll configuration described above. For example, Fig. 6 shows a tamper transfer configuration that includes a tamper member 144 that moves back and forth in the directions shown. The tamper member 144 moves back and forth to periodically engage the second surface 140 of the second substrate 106 and move the first surface 138 of the second substrate 106 into contact with one of the waistbands 102 on the anvil roll 112. In another example, shown in Fig. 7, a transfer roll 146 is placed in contact with the second surface 138 of the second substrate 106. An outer surface 146 of the transfer roll 144 and the outer surface 124 of the anvil roll 112 may be separated by a distance D shown in Fig. 7. The distance D may be greater than or equal to the thickness second substrate 106, and the distance D may be less than or equal to the combined thickness of the second substrate 106 and the individual waistbands 102. As such, the second substrate 106 alone can pass between the anvil roll 112 and the transfer roll 144 with relatively low interference. However, the combination of the second substrate 106 and individual waistband 102 between the anvil roll 112 and transfer roll 144 will result in some interference, causing the second substrate 106 and waistband 102 to be pressed together and transfer the waistband 102 from the anvil roll 112 onto the second substrate 106.

As previously mentioned, the speed of the second substrate 106 may be faster than the speed of the first substrate 104, and consequently, faster than the speed at which the individual waistbands 102 travel while located on the outer surface 124 of the anvil roll 112. As such, the mismatched speeds between the second substrate 106 and the waistbands 102 allow the second substrate 102 to advance a desired distance in the machine direction MD between waistband applications. In turn, the waistbands 102 are spaced apart from each other in the machine direction along the length of the second substrate 106.

Although the above description relates mainly to the application of elastic waistbands to diapers, it is to be appreciated that the methods and apparatuses disclosed herein can be adapted to perform other processes. For example, the methods and apparatus of the present disclosure could be used with multiple material streams that are combined after the spreader mechanism and before the cutter. In another scenario, if it is desirable to apply a wrinkled substrate on an absorbent article, the spreading operation could also be carried out opposite to the above description, causing the first substrate to wrinkle instead of stretch. It should also be appreciated that although the above description can be used to deliver one patch per diaper or absorbent article, multiple patches per diaper or absorbent article could also be delivered by changing the speed of the anvil roll and/or substrates.

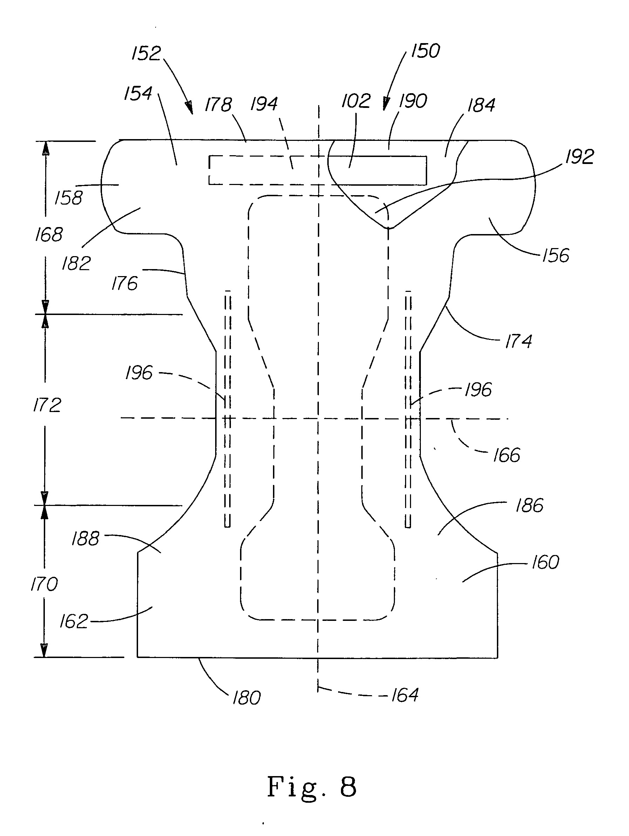

A number of different products may be manufactured in accordance with the methods described herein. For the purposes of a specific illustration, Fig. 8 shows one example of a disposable absorbent article 150 in the form of a diaper 152 that may include an elastic waist feature 102 attached thereto in accordance with the present disclosure. In particular, Fig. 8 is a plan view of one embodiment of a diaper 152 including a chassis 154 shown in a flat, unfolded condition, with the portion of the diaper 152 that faces a wearer oriented towards the viewer. A portion of the chassis structure is cut-away in Fig. 8 to more clearly show the construction of and various features that may be included in embodiments of the diaper.

As shown in Fig. 8, the diaper 152 includes a chassis 154 having a first ear 156, a second ear 158, a third ear 160, and a fourth ear 162. To provide a frame of reference for the present discussion, the chassis is shown with a longitudinal axis 164 and a lateral axis 166. The chassis 154 is shown as having a first waist region 168, a second waist region 170, and a crotch region 172 disposed intermediate the first and second waist regions. The periphery of the diaper is defined by a pair of longitudinally extending side edges 174, 176; a first outer edge 178 extending laterally adjacent the first waist region 168; and a second outer edge 180 extending laterally adjacent the second waist region 170.

As shown in Fig. 8, the chassis 154 includes an inner, body-facing surface 182, and an outer, garment-facing surface 184. A portion of the chassis structure is cut-away in Fig. 8 to more clearly show the construction of and various features that may be included in the diaper. As shown in Fig. 8, the chassis 154 of the diaper 152 may include an outer covering layer 186 including a topsheet 188 and a backsheet 190. An absorbent core 192 may be disposed between a portion of the topsheet 188 and the backsheet 190. As discussed in more detail below, any one or more of the regions may be stretchable and may include an elastomeric material or laminate as described herein. As such, the diaper 152 may be configured to adapt to a specific wearer's anatomy upon application and to maintain coordination with the wearer's anatomy during wear.

As previously mentioned, the chassis 154 of the diaper 152 may include the backsheet 190, shown for example, in Fig. 8. In some embodiments, the backsheet is configured to prevent exudates absorbed and contained within the chassis from soiling articles that may contact the diaper, such as bedsheets and undergarments. Some embodiments of the backsheet may be fluid permeably, while other embodiments may be impervious to liquids (e.g., urine) and comprises a thin plastic film. In some embodiments, the plastic film includes a thermoplastic film having a thickness of about 0.012 mm (0.5 mil) to about 0.051 mm (2.0 mils). Some backsheet films may include those manufactured by Tredegar Industries Inc. of Terre Haute, Ind, and sold under the trade names X15306, X10962, and X10964. Other backsheet materials may include breathable materials that permit vapors to escape from the diaper while still preventing exudates from passing through the backsheet. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, Tex., under the designation EXXAIRE. Suitable breathable composite materials comprising polymer blends are available from Clopay Corporation, Cincinnati, Ohio under the name HYTREL blend P18-3097. Such breathable composite materials are described in greater detail in PCT Application No. WO 95/16746, published on Jun. 22, 1995 in the name of E. I. DuPont and U.S. Pat. No. 5,865,823, issued on Feb. 2, 1999 to Curro, both of which are hereby incorporated by reference herein. Other breathable backsheets including nonwoven webs and apertured formed films are described in U.S. Pat. No. 5,571,096 issued to Dobrin et al. on Nov. 5, 1996; and U.S. Pat. No. 6,573, 423 issued to Herrlein et al. on June 3, 2003.

The backsheet 190, or any portion thereof, may be stretchable in one or more directions. In one embodiment, the backsheet may comprise a structural elastic-like film ("SELF") web. Embodiments of SELF webs are more completely described in U.S. Pat. No. 5,518,801, entitled "Web Materials Exhibiting Elastic-Like Behavior," which issued to Chappell et al. on May 21, 1996, U.S. Pat. No. 5,723,087, entitled "Web Materials Exhibiting Elastic-Like Behavior," which issued to Chappell et al. on Mar. 3, 1998; U.S. Pat. No. 5,691,035, entitled "Web Materials Exhibiting Elastic-Like Behavior," which issued to Chappell et al. on Nov. 25, 1997; U.S. Pat. No. 5,891,544, entitled "Web Materials Exhibiting Elastic-Like Behavior," which issued to Chappell et al. on Apr. 6, 1999; U.S. Pat. No. 5,916,663, entitled "Web Materials Exhibiting Elastic-Like Behavior," which issued to Chappell et al. on Jun. 29, 1999; and U.S. Pat. No. 6,027,483, entitled "Web Materials Exhibiting Elastic-Like Behavior," which issued to Chappell et al. on Fe. 22, 2000. In some embodiments, the backsheet may comprise elastomeric films, foams, strands, nonwovens, or combinations of these or other suitable materials with nonwovens or synthetic films. Additional embodiments include backsheets that comprise a stretch nonwoven material; an elastomeric film in combination with an extensible nonwoven; an elastomeric nonwoven in combination with an extensible film; and/or combinations thereof. Details on such backsheet embodiments are more completely described in U.S. Publication No. 2007/0287348 U.S. Publication No. 2007/0287982; and U.S. Publication No. 2007/0287983.

The backsheet 190 may be joined with the topsheet 188, the absorbent core 192, and/or other elements of the diaper 152 in various ways. For example, the backsheet may be connected with a uniform continuous layer of adhesive, a patterned layer of adhesive, or an array of separate lines, spirals, or spots of adhesive. One embodiment utilizes an open pattern network of filaments of adhesive as disclosed in U.S. Pat. No. 4,573,986, entitled "Disposable Waste-Containment Garment," which issued to Minetola et al. on Mar. 4, 1986. Other embodiments utilize several lines of adhesive filaments which are swirled into a spiral pattern, as is illustrated by the apparatus and methods shown in U.S. Pat. No. 3,911,173, issued to Sprague, Jr. on Oct. 7, 1975; U.S. Pat. No. 4,785,996, issued to Ziecker, et al. on Nov. 22, 1988; and U.S. Pat. No. 4,842,666 issued to Werenicz on Jun. 27, 1989. Adhesives may include those manufactured by H. B. Fuller Company of St. Paul, Minn. and marketed as HL-1620 and HL-1358-XZP. In some embodiments, the backsheet is connected with heat bonds, pressure bonds, ultrasonic bonds, dynamic mechanical bonds, or any other suitable attachment means or a combination thereof.

The topsheet 188 may be joined to the backsheet 190, the absorbent core 192, and/or other elements of the diaper 152 in various ways. For example, the topsheet 188 may be connected in ways described above with respect to joining the backsheet 190 to other elements of the diaper 152. In one embodiment, the topsheet 188 and the backsheet 190 are joined directly to each other along the outer edge of the chassis. In another embodiment, the topsheet and the backsheet are joined directly to each other in some locations and are indirectly joined together in other locations. Other topsheet and backsheet connection configurations are described in more detail in U.S. Publication No. 2007/0287981 entitled "Absorbent Article Having a Multifunctional Containment Member," filed on June 7, 2006,

The topsheet 188 may be constructed to be compliant, soft feeling, and non-irritating to the wearer's skin. Further, all or at least a portion of the topsheet 140 may be liquid pervious, permitting liquid to readily penetrate therethrough. As such, the topsheet may be manufactured from a wide range of materials, such as porous foams; reticulated foams; apertured nonwovens or plastic films; or woven or nonwoven webs of natural fibers (e.g., wood or cotton fibers), synthetic fibers (e.g., polyester or polypropylene fibers), or a combination of natural and synthetic fibers. If the absorbent assemblies include fibers, the fibers may be spunbonded, carded, wet-laid, meltblown, hydroentangled, or otherwise processed as is known in the art. One example of a topsheet including a web of staple length polypropylene fibers is manufactured by Veratec, Inc., a Division of International Paper Company, of Walpole, Mass. under the designation P-8.

Examples of formed film topsheets are described in U.S. Pat. No. 3,929,135, entitled "Absorptive Structures Having Tapered Capillaries," which issued to Thompson on Dec. 30, 1975; U.S. Pat. No. 4,324,246, entitled "Disposable Absorbent Article Having A Stain Resistant Topsheet," which issued to Mullane, et al. on Apr. 13, 1982; U.S. Pat. No. 4,342,314, entitled "Resilient Plastic Web Exhibiting Fiber-Like Properties," which issued to Radel, et al. on Aug. 3, 1982; U.S. Pat. No. 4,463,045, entitled "Macroscopically Expanded Three-Dimensional Plastic Web Exhibiting Non-Glossy Visible Surface and Cloth-Like Tactile Impression," which issued to Ahr, et al. on Jul. 31, 1984; and U.S. Pat. No. 5,006,394, entitled "Multilayer Polymeric Film," which issued to Baird on Apr. 9, 1991. Other topsheets may be made in accordance with U.S. Pat. Nos. 4,609,518 and 4,629,643, which issued to Curro et al. on Sep. 2, 1986, and Dec. 16, 1986, respectively. Such formed films are available from The Procter & Gamble Company of Cincinnati, Ohio as "DRI-WEAVE" and from Tredegar Corporation of Terre Haute, Ind. as "CLIFF-T."

In some embodiments, the topsheet 188 is made of a hydrophobic material or is treated to be hydrophobic in order to isolate the wearer's skin from liquids contained in the absorbent core. If the topsheet is made of a hydrophobic material, at least the upper surface of the topsheet may be treated to be hydrophilic so that liquids will transfer through the topsheet more rapidly. This diminishes the likelihood that body exudates will flow off the topsheet rather than being drawn through the topsheet and being absorbed by the absorbent core. The topsheet can be rendered hydrophilic by treating it with a surfactant or by incorporating a surfactant into the topsheet. Suitable methods for treating the topsheet with a surfactant include spraying the topsheet material with the surfactant and immersing the material into the surfactant. A more detailed discussion of such a treatment and hydrophilicity is contained in U.S. Pat. No. 4,988,344, entitled "Absorbent Articles with Multiple Layer Absorbent Layers," which issued to Reising, et al. on Jan. 29, 1991, and U.S. Pat. No. 4,988,345, entitled "Absorbent Articles with Rapid Acquiring Absorbent Cores," which issued to Reising on Jan. 29, 1991. A more detailed discussion of some methods for incorporating surfactant in the topsheet can be found in U.S. Statutory Invention Registration No. H1670, which was published on Jul. 1, 1997, in the names of Aziz et al..

In some embodiments, the topsheet 188 may include an apertured web or film that is hydrophobic. This may be accomplished eliminating the hydrophilizing treatment step from the production process and/or applying a hydrophobic treatment to the topsheet, such as a polytetrafluoroethylene compound like SCOTCHGUARD or a hydrophobic lotion composition, as described below. In such embodiments, the apertures may be large enough to allow the penetration of aqueous fluids like urine without significant resistance. A more detailed discussion of various apertured topsheets can be found in U.S. Pat. No. 5,342,338, entitled "Disposable Absorbent Article for Low-Viscosity Fecal Material," which issued to Roe on Aug. 30, 1994; U.S. Pat. No. 5,941,864, entitled "Disposable Absorbent Article having Improved Fecal Storage," which issued to Roe on Aug. 24, 1999; U.S. Pat. No. 6,010,491, entitled "Viscous Fluid Bodily Waste Management Article," which issued to Roe et al. on Jan. 4, 2000; and U.S. Pat. No. 6,414,215, entitled "Disposable Absorbent Article having Capacity to Store Low-Viscosity Fecal Material," which issued to Roe on July 2, 20002.

Any portion of the topsheet 188 may be coated with a lotion, such as topsheets described in U.S. Pat. No. 5,607,760, entitled "Disposable Absorbent Article Having A Lotioned Topsheet Containing an Emollient and a Polyol Polyester Immobilizing Agent," which issued to Roe on Mar. 4, 1997; U.S. Pat. No. 5,609,587, entitled "Diaper Having A Lotion Topsheet Comprising A Liquid Polyol Polyester Emollient And An Immobilizing Agent," which issued to Roe on Mar. 11, 1997; U.S. Pat. No. 5,635,191, entitled "Diaper Having A Lotioned Topsheet Containing A Polysiloxane Emollient," which issued to Roe et al. on Jun. 3, 1997; U.S. Pat. No. 5,643,588, entitled "Diaper Having A Lotioned Topsheet," which issued to Roe et al. on Jul. 1, 1997; and U.S. Pat. No. 6,498,284, entitled "Disposable Absorbent Article with a Skin Care Composition on an Apertured Top Sheet," which issued to Roe on Dec. 24, 2002. The lotion may function alone or in combination with another agent as the hydrophobizing treatment described above. The topsheet may also include or be treated with antibacterial agents, some examples of which are disclosed in PCT Publication No. WO 95/24173 entitled "Absorbent Articles Containing Antibacterial Agents in the Topsheet For Odor Control," which was published on Sep. 14, 1995, in the name of Theresa Johnson. Further, the topsheet, the backsheet, or any portion of the topsheet or backsheet may be embossed and/or matte finished to provide a more cloth like appearance.

Embodiments of the absorbent article may also include pockets for receiving and containing waste, spacers which provide voids for waste, barriers for limiting the movement of waste in the article, compartments or voids which accept and contain waste materials deposited in the diaper, and the like, or any combinations thereof. Examples of pockets and spacers for use in absorbent products are described in U.S. Pat. No. 5,514,121 issued to Roe et al. on May 7, 1996, entitled "Diaper Having Expulsive Spacer"; U.S. Pat. No. 5,171,236 issued to Dreier et al on Dec. 15, 1992, entitled "Disposable Absorbent Article Having Core Spacers"; U.S. Pat. No. 5,397,318 issued to Dreier on Mar. 14, 1995, entitled "Absorbent Article Having A Pocket Cuff"; U.S. Pat. No. 5,540,671 issued to Dreier on Jul. 30, 1996, entitled "Absorbent Article Having A Pocket Cuff With An Apex"; and PCT Application WO 93/25172 published Dec. 3, 1993, entitled "Spacers For Use In Hygienic Absorbent Articles And Disposable Absorbent Articles Having Such Spacer"; and U.S. Pat. No. 5,306,266, entitled "Flexible Spacers For Use In Disposable Absorbent Articles", issued to Freeland on Apr. 26, 1994. Examples of compartments or voids are disclosed in U.S. Pat. No. 4,968,312, entitled "Disposable Fecal Compartmenting Diaper", issued to Khan on Nov. 6, 1990; U.S. Pat. No. 4,990,147, entitled "Absorbent Article With Elastic Liner For Waste Material Isolation", issued to Freeland on Feb. 5, 1991; U.S. Pat. No. 5,062,840, entitled "Disposable Diapers", issued to Holt et al on Nov. 5, 1991; U.S. Pat. No. 6,482,191 entitled "Elasticated Topsheet with an Elongate Slit Opening," issued to Roe et al. on Nov. 19, 2002; and U.S. Pat. No. 5,269,755 entitled "Trisection Topsheets For Disposable Absorbent Articles And Disposable Absorbent Articles Having Such Trisection Topsheets", issued to Freeland et al. on Dec. 14, 1993. Examples of suitable transverse barriers are described in U.S. Pat. No. 5,554,142 entitled "Absorbent Article Having Multiple Effective Height Transverse Partition" issued Sep. 10, 1996 in the name of Dreier et al.; PCT Patent WO 94/14395 entitled "Absorbent Article Having An Upstanding Transverse Partition" published Jul. 7, 1994 in the name of Freeland, et al., and U.S. Pat No. 5,653,703 Absorbent Article Having Angular Upstanding Transverse Partition, issued Aug. 5, 1997 to Roe, et al.. In addition to or in place of the voids, pockets and barriers, described above, embodiments of the absorbent article may also include a waste management element capable of effectively and efficiently accepting, storing and/or immobilizing viscous fluid bodily waste, such as runny feces, such as described in U.S. Pat. No. 6,010,491 issued to Roe et al. on Jan. 4, 2000.

The absorbent core 192 may include absorbent material that is generally compressible, conformable, non-irritating to the wearer's skin, and capable of absorbing and retaining liquids such as urine and other body exudates. The absorbent core 192 can also be manufactured in a wide variety of sizes and shapes (e.g., rectangular, hourglass, T-shaped, asymmetric, etc.). The absorbent core may also include a wide variety of liquid-absorbent materials commonly used in disposable diapers and other absorbent articles. In one example, the absorbent core includes comminuted wood pulp, which is generally referred to as airfelt. Examples of other absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

It is to be appreciated that the configuration and construction of the absorbent core 192 may be varied (e.g., the absorbent core(s) or other absorbent structure(s) may have varying caliper zones, a hydrophilic gradient, a superabsorbent gradient, or lower average density and lower average basis weight acquisition zones; or may comprise one or more layers or structures).

Exemplary absorbent structures are described in U.S. Pat. No. 4,610,678, entitled "High-Density Absorbent Structures," which issued to Weisman et al. on Sep. 9, 1986; U.S. Pat. No. 4,673,402, entitled "Absorbent Articles With Dual-Layered Cores," which issued to Weisman et al. on Jun. 16, 1987; U.S. Pat. No. 4,834,735, entitled "High Density Absorbent Members Having Lower Density and Lower Basis Weight Acquisition Zones," which issued to Alemany et al. on May 30, 1989; U.S. Pat. No. 4,888,231, entitled "Absorbent Core Having A Dusting Layer," which issued to Angstadt on Dec. 19, 1989; U.S. Pat. No. 5,137,537, entitled "Absorbent Structure Containing Individualized, Polycarboxylic Acid Crosslinked Wood Pulp Cellulose Fibers," which issued to Herron et al. on Aug. 11, 1992; U.S. Pat. No. 5,147,345, entitled "High Efficiency Absorbent Articles For Incontinence Management," which issued to Young et al. on Sep. 15, 1992; U.S. Pat. No. 5,342,338, entitled "Disposable Absorbent Article For Low-Viscosity Fecal Material," issued to Roe on Aug. 30, 1994; U.S. Pat. No. 5,260,345, entitled "Absorbent Foam Materials For Aqueous Body Fluids and Absorbent Articles Containing Such Materials," which issued to DesMarais et al. on Nov. 9, 1993; U.S. Pat. No. 5,387,207, entitled "Thin-Until-Wet Absorbent Foam Materials For Aqueous Body Fluids And Process For Making Same," which issued to Dyer et al. on Feb. 7, 1995; and U.S. Pat. No. 5,650,222, entitled "Absorbent Foam Materials For Aqueous Fluids Made From high Internal Phase Emulsions Having Very High Water-To-Oil Ratios," which issued to DesMarais et al. on Jul. 22, 1997.

The absorbent core 192 may also have a multiple layered construction. A more detailed discussion of various types of multi-layered absorbent cores can be found in U.S. Pat. No. 5,669,894, entitled "Absorbent Members for Body Fluids having Good Wet Integrity and Relatively High Concentrations of Hydrogel-forming Absorbent Polymer," issued to Goldman et al. on Sept. 23, 1997; U.S. Pat. No. 6,441,266, entitled "Absorbent Members for Body Fluids using Hydrogel-forming Absorbent Polymer," issued to Dyer et al. on Aug. 26, 2002; U.S. Pat. No. 5,562,646, entitled "Absorbent Members for Body Fluids having Good Wet Integrity and Relatively High Concentrations of Hydrogel-forming Absorbent Polymer having High Porosity," issued to Goldman et al. on Oct. 10, 1996; European Pat. No. EP0565606B1, published on Mar. 8, 1995; U.S. Pat. Publication No. 2004/0162536A1 published Aug. 19, 2004; U.S. Pat. Publication No. 2004/0167486A1 published on Aug. 26, 2004; and PCT Publication No. WO 2006/015141 published on Feb. 9, 2006. In some embodiments, the absorbent article includes an absorbent core that is stretchable. In such a configuration, the absorbent core may be adapted to extend along with other materials of the chassis in longitudinal and/or lateral directions. The absorbent core can also be connected with the other components of the chassis various ways. For example, the diaper may include a "floating core" configuration or a "bucket" configuration wherein the diaper includes an anchoring system that can be configured to collect forces tending to move the article on the wearer. Such an anchoring system can also be configured to anchor itself to a body of a wearer by contacting various parts of the body. In this way, the anchoring system can balance the collected moving forces with holding forces obtained from the anchoring. By balancing the collected moving forces with the obtained holding forces, the anchoring system can at least assist in holding the disposable wearable absorbent article in place on a wearer. A more detailed discussion of various floating and/or bucket core configurations can be found in U.S. provisional patent application number 60/811,700, entitled "Absorbent Article Having a Multifunctional Containment Member," filed on June 7, 2006; U.S. Application No. 11/599,851; and U.S. Application No. 11/599,862.

The elastic waist feature 102 shown in Fig. 8 is in the form of a waist band 194 and may provide improved fit and waste containment. The elastic waist feature 102 may be configured to elastically expand and contract to dynamically fit the wearer's waist. The elastic waist feature 102 can be incorporated into the diaper in accordance with the methods discussed herein and may extend at least longitudinally outwardly from the absorbent core 192 and generally form at least a portion of the first and/or second outer edges 178, 180 of the diaper 152. In addition, the elastic waist feature may extend laterally to include the ears. While the elastic waist feature 102 or any constituent elements thereof may comprise one or more separate elements affixed to the diaper, the elastic waist feature may be constructed as an extension of other elements of the diaper, such as the backsheet 190, the topsheet 188, or both the backsheet and the topsheet. In addition, the elastic waist feature 102 may be disposed on the outer, garment-facing surface 184 of the chassis 140; the inner, body-facing surface 182; or between the inner and outer facing surfaces.

The elastic waist feature 102 may be constructed in a number of different configurations including those described in U.S. Patent No. 7432413, filed on December 16, 2005; U.S. Patent Publication No. 2007/0142798 on December 16, 2005; and U.S. Publication No. 2007/0142798 filed on November 15, 2006.

Although the first and second ears 156, 158 as well as the third and fourth ears 160, 162 shown in Fig. 8 are illustrated as being integrally formed with the chassis 140, it is to be appreciated that other embodiments may include ears that are discrete elements connected with the chassis. In some embodiments, the ears are configured to be stretchable, and in some embodiments, it may be preferable to have elastically stretchable ears. As discussed in more detail below, the ears may also include one or more fastener elements adapted to releasably connect with each other and/or other fastener elements on the chassis. A more detailed discussion of stretchable ears can be found in U.S. Pat. No. 4,857,067, entitled "Disposable Diaper Having Shirred Ears" issued to Wood, et al. on Aug. 15, 1989; U.S. Pat. No. 5,151,092 issued to Buell et al. on Sep. 29, 1992; U.S. Pat. No. 5,674,216 issued to Buell et al. on Oct. 7, 1997; U.S. Pat. No. 6,677,258 issued to Carroll et al. on Jan. 13, 2004; U.S. Pat. No. 4,381,781 issued to Sciaraffa, et al. on May 3, 1983; U.S. Pat. No. 5,580,411 entitled "Zero Scrap Method For Manufacturing Side Panels For Absorbent Articles" issued to Nease, et al. on December 3, 1996; and U.S. Patent No. 6,004,306 entitled "Absorbent Article With Multi-Directional Extensible Side Panels" issued to Robles et al. on December 21, 1999. The ears may also include various geometries and arrangements of stretch zones or elements, such as discussed in U.S. Pat. Publication No. US2005/0215972A1 published on Sept. 29, 2005, and U.S. Pat. Publication No. US2005/0215973Al published on Sept. 29, 2005.

As shown in Fig. 8, the diaper 152 may include leg cuffs 196 that may provide improved containment of liquids and other body exudates. In particular, elastic gasketing leg cuffs can provide a sealing effect around the wearer's thighs to prevent leakage. It is to be appreciated that when the diaper is worn, the leg cuffs may be placed in contact with the wearer's thighs, and the extent of that contact and contact pressure may be determined in part by the orientation of diaper on the body of the wearer. The leg cuffs 196 may be disposed in various ways on the diaper 102. For example, the leg cuffs 196 may be disposed on the outer, garment-facing surface 184 of the chassis 152; the inner, body-facing surface 182; or between the inner and outer facing surfaces. Leg cuffs 196 may also be referred to as leg bands, side flaps, barrier cuffs, or elastic cuffs. U.S. Pat. No. 3,860,003, describes a disposable diaper that provides a contractible leg opening having a side flap and one or more elastic members to provide an elasticized leg cuff (a gasketing cuff). U.S. Pat. Nos. 4,808,178 and 4,909,803, issued to Aziz et al. on Feb. 28, 1989, and Mar. 20, 1990, respectively, describe disposable diapers having "stand-up" elasticized flaps (barrier cuffs) which improve the containment of the leg regions. U.S. Pat. Nos. 4,695,278 and 4,795,454, issued to Lawson on Sep. 22, 1987, and to Dragoo on Jan. 3, 1989, respectively, describe disposable diapers having dual cuffs, including gasketing cuffs and barrier cuffs. In some embodiments, it may be desirable to treat all or a portion of the leg cuffs with a lotion, as described above. In addition to leg cuffs, diaper can also include an elastic gasketing cuff with one or more elastic strands positioned outboard of the barrier cuff. To improve waste containment, the leg cuffs may be treated with a hydrophobic surface coating, such as described in U.S. Pat. Publication No. 20060189956A1, entitled "Hydrophobic Surface Coated Light-Weight Nonwoven Laminates for Use in Absorbent Articles," published on Aug. 24, 2006.

The diaper 152 may be provided in the form of a pant-type diaper or may alternatively be provided with a re-closable fastening system, which may include fastener elements in various locations to help secure the diaper in position on the wearer. For example, fastener elements may be located on the first and second ears and may be adapted to releasably connect with one or more corresponding fastening elements located in the second waist region.

It is to be appreciated that various types of fastening elements may be used with the diaper. In one example, the fastening elements include hook & loop fasteners, such as those available from 3M or Velcro Industries. In other examples, the fastening elements include adhesives and/or tap tabs, while others are configured as a macrofastener or hook (e.g., a MACRO or "button-like" fastener). Some exemplary fastening elements and systems are disclosed in U.S. Pat. No. 3,848,594, entitled "Tape Fastening System for Disposable Diaper," which issued to Buell on Nov. 19, 1974; U.S. Pat. No. B1 4,662,875, entitled "Absorbent Article," which issued to Hirotsu et al. on May 5, 1987; U.S. Pat. No. 4,846,815, entitled "Disposable Diaper Having An Improved Fastening Device," which issued to Scripps on Jul. 11, 1989; U.S. Pat. No. 4,894,060, entitled "Disposable Diaper With Improved Hook Fastener Portion," which issued to Nestegard on Jan. 16, 1990; U.S. Pat. No. 4,946,527, entitled "Pressure-Sensitive Adhesive Fastener And Method of Making Same," which issued to Battrell on Aug. 7, 1990; and U.S. Pat. No. 5,151,092, issued to Buell on Sep. 29, 1992; and U.S. Pat. No. 5,221,274, which issued to Buell on Jun. 22, 1993. Additional examples of fasteners and/or fastening elements are discussed in U.S. Pat. Nos. 6,251,097 and 6,432,098; U.S. Publication No. 2007/0078427, entitled, "Anti-Pop Open Macrofasteners" filed on September 30, 2005; and U.S. Patent 7799006 , entitled, "A Fastening System Having Multiple Engagement Orientations", filed on September 30, 2005. Other fastening systems are described in more detail in U.S. Pat. No. 5,595,567 issued to King et al. on Jan. 21, 1997 and U.S. Pat. No. 5,624,427 issued to Bergman et al. on Apr. 29, 1997, both of which are entitled "Nonwoven Female Component For Refastenable Fastening Device." Yet other fastening systems are described in U.S. Pat. Nos. 5,735,840 and 5,928,212, both of which issued to Kline et al. and are entitled "Disposable Diaper With Integral Backsheet Landing Zone,". The fastening system may also provide a means for holding the article in a disposal configuration as disclosed in U.S. Pat. No. 4,963,140, which issued to Robertson et al. on Oct. 16, 1990.

It is also to be appreciated that diapers 152 according the present disclosure may be constructed with various types of the previously described materials that allow the entire chassis 140 or portions of the chassis, such as the ears 156, 158, 160, 162, crotch region 172, and/or waist regions 168, 170 to stretch. It is to be appreciated that the entire chassis or portions of the chassis can be configured to stretch in longitudinal directions, lateral directions, or both (i.e. biaxial stretch). In some embodiments, the chassis may include regions of longitudinal stretch, regions of lateral stretch, and/or regions of biaxial stretch. For example, in some embodiments, the entire length of the crotch region 172 is adapted to stretch in longitudinal and/or lateral directions. In other embodiments, opposing end regions of the crotch region 172 is the only portion of the chassis 140 that is longitudinally and/or laterally stretchable. In yet other embodiments, central or proximal regions of the crotch region are the only portions of the chassis 140 that are longitudinally and/or laterally stretchable. In such example configurations, the crotch region or sub-regions thereof may comprise a different material than that of the remainder of the chassis 140, may have been subjected to a different treatment (e.g. SELFing, mechanical ringrolling), or a combination thereof. References disclosing structural elastic-like film ("SELF") materials are discussed above. The chassis may also be constructed with a "zero strain" stretch laminate. Zero strain stretch laminates can be made by bonding an elastomer to a nonwoven while both are in an unstrained state. A more detailed discussion of zero strain laminates can be found in U.S. Pat. No. 5,156,793, entitled "Method for Incrementally Stretching Zero Strain Stretch Laminate Web in a Non-uniform Manner to Impart a Varying Degree of Elasticity Thereto," issued to Buell et al. on Oct. 20, 1992. In another example, the chassis may be constructed with "live stretch," which may include stretching elastic and bonding the stretched elastic to a nonwoven. After bonding the stretched elastic is released causing it to contract, resulting in a "corrugated" nonwoven. A more detailed discussion of "live stretch" can be found in U.S. Pat. No. 4,720,415 to Vander Wielen, et al., issued Jan. 19,1988 and U.S. Pat. No. 7,028,735 to Schneider et al. issued on April 18, 2006.

## Claims

1. A method for applying elastic components to a moving substrate, the method comprising the steps of:
continuously feeding a first substrate (104) of material in a machine direction at a first speed, the first substrate (104) having a first surface (120) disposed opposite of a second surface (122);
continuously feeding a second substrate (106) of material in the machine direction at a second speed, the second substrate (106) having a first surface (138) disposed opposite of a second surface (140);
then stretching the first substrate (104) in a cross direction;
rotating an anvil roll (112) at a first rotational speed wherein an outer surface (124) of the anvil roll (112) travels at the first speed;
then feeding the first substrate (104) onto the anvil roll (112) such that the first surface (120) of the first substrate (104) is disposed on the outer surface (124) of the anvil roll (112);
maintaining the cross direction stretch of the first substrate (104) while disposed on the outer surface (124) of the anvil roll (112);
then applying glue (118) to the second surface (122) of the first substrate (104);
then cutting the stretched first substrate (104) on the anvil roll (112) into discrete stretched elastic strips (102); and
individually transferring the discrete stretched elastic strips (102) from the anvil roll (112) onto the first surface (138) of the second substrate (106) such that the glue (118) adheres the discrete stretched elastic strips (102) to the second substrate (106), and wherein the discrete stretched strips (102) are displaced from each other in the machine direction along a length of the second substrate (106).

2. The method of claim 1, wherein the step of stretching the first substrate comprises stretching the first substrate (104) in the cross direction with canted rolls (108).

3. The method of claim 1, wherein the step of stretching the first substrate comprises stretching the first substrate (104) in the cross direction with diverging conveyors.

4. The method according to any of the preceding claims, wherein the step of transferring the discrete stretched elastic strips from the anvil roll onto the first surface of the second substrate further comprises the step of intermittently moving the second substrate (106) into contact with individual discrete stretched elastic strips (102) on the anvil roll (112).

5. The method of claim 4, wherein the step of transferring the discrete stretched elastic strips from the anvil roll onto the first surface of the second substrate further comprises passing the second surface (140) of the second substrate (106) adjacent a rotating bump roll (136) having an outer surface with a protrusion (142), wherein the protrusion (142) intermittently engages the second surface (140) of the second substrate (106) to move the first surface (138) of the second substrate (106) into contact with an individual discrete stretched elastic strip (102).

6. The method of claim 4, wherein the step of transferring the discrete stretched elastic strips from the anvil roll onto the first surface of the second substrate further comprises passing the second surface (140) of the second substrate (106) adjacent a tamper member (144) that intermittently engages the second surface (140) of the second substrate (106) to move the first surface (138) of the second substrate (106) into contact with an individual discrete stretched elastic strip (102).

7. The method according to any of claims 1 to 3, wherein the step of transferring the discrete stretched elastic strips from the anvil roll onto the first surface of the second substrate further comprises advancing the second substrate (106) between the anvil roll (112) and a transfer roll (144), wherein a maximum distance (D) between outer surfaces (124, 146) of the anvil roll (112) and the transfer roll (124) is less than or equal to a combination of a thickness of the second substrate (106) and a thickness of the discrete stretched elastic strips (102).

8. The method according to any of the preceding claims, wherein the second substrate (106) comprises a backsheet of a diaper and the discrete stretched elastic strips comprise waistbands.

9. The method according to any of the preceding claims, wherein the first surface of the second substrate (106) comprises an outer surface of the backsheet.

10. The method according to any of the preceding claims, wherein the second speed is greater than the first speed.

## Patentansprüche

1. Verfahren zum Auftragen elastischer Bestandteile auf ein sich bewegendes Substrat, wobei das Verfahren folgende Schritte umfasst:
das ununterbrochene Zuführen eines ersten Substrats (104) an Material in eine Maschinenlaufrichtung bei einer ersten Geschwindigkeit, wobei das erste Substrat (104) eine erste Fläche (120) aufweist, die einer zweiten Fläche (122) entgegengesetzt angeordnet ist;
das ununterbrochene Zuführen eines zweiten Substrats (106) an Material in eine Maschinenlaufrichtung bei einer zweiten Geschwindigkeit, wobei das zweite Substrat (106) eine erste Fläche (138) aufweist, die einer zweiten Fläche (140) entgegengesetzt angeordnet ist;
und das anschließende Dehnen des ersten Substrats (104) in einer Querrichtung;
das Drehen einer Ambosswalze (112) bei einer ersten Drehgeschwindigkeit, wobei eine erste Außenfläche (124) der Ambosswalze (112) mit der ersten Geschwindigkeit läuft;
das anschließende Zuführen des ersten Substrats (104) auf die Ambosswalze (112) solcherart, dass die erste Fläche (120) des ersten Substrats (104) auf der Außenfläche (124) der Ambosswalze (112) angeordnet wird;
das Beibehalten der Dehnung in Querrichtung des ersten Substrats (104),
während dieses auf der Außenfläche (124) der Ambosswalze (112) angeordnet wird;
das anschließende Auftragen von Klebstoff (118) auf die zweite Fläche (122) des ersten Substrats (104);
das anschließende Schneiden des gedehnten ersten Substrats (104) auf der Ambosswalze (112) in separate gedehnte elastische Streifen (102); und
das einzelne Überführen der separaten gedehnten elastischen Streifen (102) von der Ambosswalze (112) auf die erste Fläche (138) des zweiten Substrats (106) solcherart, dass der Klebstoff (118) die separaten gedehnten elastischen Streifen (102) mit dem zweiten Substrat (106) verbindet, und wobei die separaten gedehnten Streifen(102) in Maschinenlaufrichtung entlang der Länge des zweiten Substrats (106) voneinander weg verschoben werden.

2. Verfahren nach Anspruch 1, wobei der Schritt des Dehnens des ersten Substrats das Dehnen des ersten Substrats (104) in Querrichtung mit abgeschrägten Walzen (108) umfasst.

3. Verfahren nach Anspruch 1, wobei der Schritt des Dehnens des ersten Substrats das Dehnen des ersten Substrats (104) in Querrichtung mit auseinanderlaufenden Förderbändern umfasst.

4. Verfahren nach einem der vorstehenden Ansprüche, wobei der Schritt des Übertragens der separaten gedehnten elastischen Streifen von der Ambosswalze auf die erste Fläche des zweiten Substrats ferner den Schritt des unterbrochenen Inkontaktbringens des zweiten Substrats (106) mit den separaten gedehnten elastischen Streifen (102) auf der Ambosswalze (112) umfasst.

5. Verfahren nach Anspruch 4, wobei der Schritt des Übertragens der separaten gedehnten elastischen Streifen von der Ambosswalze auf die erste Fläche des zweiten Substrats ferner das Vorbeilaufen an der zweiten Fläche (140) des zweiten Substrats (106) angrenzend an eine sich drehende Höckerwalze (136) umfasst, deren Außenfläche einen Vorsprung (142) aufweist, wobei der Vorsprung (142) mit Unterbrechungen in die zweite Fläche (140) des zweiten Substrats (106) eingreift, um die erste Fläche (138) des zweiten Substrats (106) mit einem separaten gedehnten elastischen Streifen (102) in Kontakt zu bringen.

6. Verfahren nach Anspruch 4, wobei der Schritt des Übertragens der separaten gedehnten elastischen Streifen von der Ambosswalze auf die erste Fläche des zweiten Substrats ferner das Vorbeilaufen an der zweiten Fläche (140) des zweiten Substrats (106) angrenzend an ein sich drehendes Stampferelement (144) umfasst, das mit Unterbrechungen in die zweite Fläche (140) des zweiten Substrats (106) eingreift, um die erste Fläche (138) des zweiten Substrats (106) mit einem separaten gedehnten elastischen Streifen (102) in Kontakt zu bringen.

7. Verfahren nach einem der Ansprüche 1 bis 3, wobei der Schritt des Übertragens der separaten gedehnten elastischen Streifen von der Ambosswalze auf die erste Fläche des zweiten Substrats ferner das Voranbewegen des zweiten Substrats (106) zwischen der Ambosswalze (112) und einer Übertragungswalze (144) umfasst, wobei ein maximaler Abstand (D) zwischen den Außenflächen (124, 146) der Ambosswalze (112) und der Übertragungswalze (124) kleiner oder gleich einer Kombination aus einer Dicke des zweiten Substrats (106) und einer Dicke der separaten gedehnten elastischen Streifen (102) ist.

8. Verfahren nach einem der vorstehenden Ansprüche, wobei das zweite Substrat (106) eine Unterschicht einer Windel umfasst und die separaten gedehnten Streifen Taillenbänder umfassen.

9. Verfahren nach einem der vorstehenden Ansprüche, wobei die erste Fläche des zweiten Substrats (106) eine Außenfläche der Unterschicht umfasst.

10. Verfahren nach einem der vorstehenden Ansprüche, wobei die zweite Geschwindigkeit größer ist als die erste Geschwindigkeit.

## Revendications

1. Procédé destiné à appliquer des éléments élastiques sur un substrat mobile, le procédé comprenant les étapes consistant à :
amener en continu un premier substrat (104) de matériau dans un sens machine à une première vitesse, le premier substrat (104) ayant une première surface (120) disposée à l'opposé d'une seconde surface (122) ;
amener en continu un second substrat (106) de matériau dans le sens machine à une seconde vitesse, le second substrat (106) ayant une première surface (138) disposée à l'opposé d'une seconde surface (140) ;
puis étirer le premier substrat (104) dans une direction transversale ;
faire tourner un contre-rouleau (112) à une première vitesse de rotation, dans lequel une surface extérieure (124) du contre-rouleau (112) tourne à la première vitesse ;
puis amener le premier substrat (104) sur le contre-rouleau (112) de telle sorte que la première surface (120) du premier substrat (104) soit disposée sur la surface extérieure (124) du contre-rouleau (112) ;
maintenir l'étirage dans la direction transversale du premier substrat (104) tandis qu'il est disposé sur la surface extérieure (124) du contre-rouleau (112) ;
puis appliquer de la colle (118) sur la seconde surface (122) du premier substrat (104) ;
puis découper le premier substrat étiré (104) sur le contre-rouleau (112) en bandes élastiques étirées discrètes (102) ; et
transférer individuellement les bandes élastiques étirées discrètes (102) à partir du contre-rouleau (112) sur la première surface (138) du second substrat (106) de telle sorte que la colle (118) fasse adhérer les bandes élastiques étirées discrètes (102) au second substrat (106), et dans lequel les bandes étirées discrètes (102) sont décalées les unes des autres dans le sens machine sur toute la longueur du second substrat (106).

2. Procédé selon la revendication 1, dans lequel l'étape d'étirage du premier substrat comprend l'étirage du premier substrat (104) dans la direction transversale avec des rouleaux inclinés (108).

3. Procédé selon la revendication 1, dans lequel l'étape d'étirage du premier substrat comprend l'étirage du premier substrat (104) dans la direction transversale avec des convoyeurs divergents.

4. Procédé selon l'une quelconque des revendications précédentes, dans lequel l'étape de transfert des bandes élastiques étirées discrètes à partir du contre-rouleau sur la première surface du second substrat comprend en outre l'étape consistant à mettre par intermittence le second substrat (106) en contact avec les bandes élastiques étirées discrètes individuelles (102) sur le contre-rouleau (112).

5. Procédé selon la revendication 4, dans lequel l'étape de transfert des bandes élastiques étirées discrètes à partir du contre-rouleau sur la première surface du second substrat comprend en outre le passage de la seconde surface (140) du second substrat (106) à proximité d'un rouleau de bosselage rotatif (136) ayant une surface extérieure avec une saillie (142), dans lequel la saillie (142) vient par intermittence au contact de la seconde surface (140) du second substrat (106) afin de mettre la première surface (138) du second substrat (106) en contact avec une bande élastique étirée discrète individuelle (102).

6. Procédé selon la revendication 4, dans lequel l'étape de transfert des bandes élastiques étirées discrètes à partir du contre-rouleau sur la première surface du second substrat comprend en outre le passage de la seconde surface (140) du second substrat (106) à proximité d'un élément de bourrage (144) qui vient par intermittence au contact de la seconde surface (140) du second substrat (106) afin de mettre la première surface (138) du second substrat (106) en contact avec une bande élastique étirée discrète individuelle (102).

7. Procédé selon l'une quelconque des revendications 1 à 3, dans lequel l'étape de transfert des bandes élastiques étirées discrètes à partir du contre-rouleau sur la première surface du second substrat comprend en outre l'avancement du second substrat (106) entre le contre-rouleau (112) et un rouleau de transfert (144), dans lequel une distance maximale (D) entre des surfaces extérieures (124, 146) du contre-rouleau (112) et du rouleau de transfert (124) est inférieure ou égale à une combinaison d'une épaisseur du second substrat (106) et d'une épaisseur des bandes élastiques étirées discrètes (102).

8. Procédé selon l'une quelconque des revendications précédentes, dans lequel le second substrat (106) comprend une enveloppe de couche et les bandes élastiques étirées discrètes comprennent des ceintures.

9. Procédé selon l'une quelconque des revendications précédentes, dans lequel la première surface du second substrat (106) comprend une surface extérieure de l'enveloppe.

10. Procédé selon l'une quelconque des revendications précédentes, dans lequel la seconde vitesse est supérieure à la première vitesse.
